# EUROPEAN PATENT APPLICATION

(11) **EP 4 729 610 A1**
(43) Date of publication of application: **22.04.2026**
(21) Application number: 24823719.0
(22) Date of filing: 13.06.2024
(51) Int. Cl.: C12N 9/08, C12N 15/77, C12N 15/70, C12P 13/14, C07K 14/00

(54) **NOVEL ALKYLHYDROPEROXIDASE AHPD FAMILY CORE DOMAIN VARIANT, AND METHOD FOR PRODUCING L-GLUTAMIC ACID BY USING SAME**

(30) Priority: 16.06.2023 KR 20230077733; 10.06.2024 KR 20240075098
(71) Applicant: Daesang Corporation, Seoul 03130 (KR)
(72) Inventor: CHOI, Won Woo, Seoul 07789 (KR); YI, Ja Kyung, Seoul 07789 (KR); LEE, Sun Hee, Seoul 07789 (KR); KIM, Suok Su, Seoul 07789 (KR)
(74) Representative: Ipsilon
(86) International application number: PCT/KR2024/008119
(87) International publication number: WO 2024/258202

(57) **Abstract**

The present invention relates to a novel alkylhydroperoxidase AhpD family core domain variant, and a method of producing L-glutamic acid using the same. The alkylhydroperoxidase AhpD family core domain variant is obtained by substituting one or more amino acids in the amino acid sequence constituting the alkylhydroperoxidase AhpD family core domain to change the activity of the protein, and L-glutamic acid may be efficiently produced from a recombinant microorganism expressing the variant.

## Description

### Technical Field

The present invention relates to a novel alkylhydroperoxidase AhpD family core domain variant and a method of producing L-glutamic acid using the same.

### Background Art

L-glutamic acid is a typical amino acid that is produced by microbial fermentation. Monosodium L-glutamate (MSG), a salt form of L-glutamic acid, may increase the preference of foods such as meat, fish, chicken, vegetables, sauces, soups and seasonings by balancing and harmonizing the overall taste of the food, may enhance the taste of low-salt foods having a salt content reduced up to 30%, and thus is widely used as a household seasoning and a seasoning for the production of processed food.

In brief, regarding the pathway of L-glutamic acid fermentation, glucose mainly undergoes the glycolytic pathway, but a portion thereof is metabolized into two pyruvic acid molecules through the pentose phosphate pathway. Among these molecules, one molecule combines with CO₂ to form oxaloacetic acid, and the other molecule combines with acetyl CoA from pyruvic acid to form citric acid. Then, oxaloacetic acid and citric acid enter the citric acid cycle (TCA cycle) to form α-ketoglutaric acid. Here, since the TCA cycle lacks the metabolic pathway for the oxidation of α-ketoglutaric acid to succinic acid and isocitrate dehydrogenase and glutamate dehydrogenase are closely involved therein, reductive amination of α-ketoglutaric acid efficiently occurs, thus producing L-glutamic acid.

For the production of L-glutamic acid, either naturally occurring wild-type strains or mutant strains modified from the wild-type strains so as to have an increased ability to produce glutamic acid may be used. In recent years, in order to improve the efficiency of production of L-glutamic acid, there has been development of a variety of recombinant strains or mutant strains having excellent L-glutamic acid productivity by applying genetic recombination technology to microorganisms such as *Escherichia coli* and *Corynebacterium,* which are widely used in the production of useful substances such as amino acids and nucleic acids, and methods of producing L-glutamic acid using the same. In particular, there have been attempts to increase the production of L-glutamic acid by directly inducing mutations in genes such as enzymes, transcription factors and transport proteins, which are involved in the biosynthetic pathways of L-glutamic acid, or inducing mutations in promoters that regulate the expression of these genes. However, there are many types of proteins such as enzymes, transcription factors and transport proteins, which are involved directly or indirectly in the production of L-glutamic acid, and thus much research is still needed on the increase in L-glutamic acid productivity by changes in the activity of these proteins.

### [Prior Art Documents]

### [Patent Documents]

U.S. Patent No. 6,852,516
U.S. Patent No. 6,962,805

### DISCLOSURE

### Technical Problem

An object of the present invention is to provide a novel alkylhydroperoxidase AhpD family core domain variant.

Another object of the present invention is to provide a polynucleotide encoding the variant.

Still another object of the present invention is to provide a transformant comprising the variant or the polynucleotide.

Yet another object of the present invention is to provide a method of producing L-glutamic acid using the transformant.

### Technical Solution

One aspect of the present invention provides an alkylhydroperoxidase AhpD family core domain variant consisting of the amino acid sequence of SEQ ID NO: 4 in which tryptophan (W) at position 231 in the amino acid sequence of SEQ ID NO: 2 is substituted with arginine (R).

The "alkylhydroperoxidase AhpD family core domain" as used in the present invention is a component of alkylhydroperoxidase that plays a role in removing intracellular peroxides and other reactive oxygen species, and is known to play a role in regulating a stress response induced by H₂O₂. The alkylhydroperoxidase AhpD family core domain in the present invention may be a polypeptide encoded by the NCgl2349 or ahpD1 gene and having alkylhydroperoxidase AhpD family core domain activity, without being limited thereto.

Information on the nucleic acid and protein sequences of the alkylhydroperoxidase AhpD family core domain is available from known sequence databases (e.g., GenBank, UniProt).

According to one specific example of the present invention, the alkylhydroperoxidase AhpD family core domain may be encoded by the nucleotide sequence of SEQ ID NO: 1 and may consist of the amino acid sequence of SEQ ID NO: 2.

The nucleotide sequence or amino acid sequence of the alkylhydroperoxidase AhpD family core domain according to the present invention may comprise a nucleotide sequence or amino acid sequence having at least 70%, 75%, 80%, 85%, 90%, 95%, 96%, 97%, 98%, 99% or 100% homology or identity to the nucleotide sequence of SEQ ID NO: 1 or the amino acid sequence of SEQ ID NO: 2. As used herein, the term "homology" or "identity" means the percentage of identity between two sequences, which is determined by aligning the reference nucleotide sequence or amino acid sequence and any other nucleotide sequence or amino acid sequence to correspond to each other as much as possible and analyzing the aligned sequences.

According to one embodiment of the present invention, the alkylhydroperoxidase AhpD family core domain or the gene encoding the same may be derived from wild-type *Corynebacterium glutamicum.*

As used in the present invention, the term "variant" refers to a protein that has an amino acid sequence different from the original amino acid sequence of the protein due to mutation in the nucleotide sequence of the gene encoding the protein. Specifically, the mutation in the gene sequence is caused by the substitution, insertion, deletion or the like of one or more bases or nucleotides in the sequence of the gene, and a polypeptide or protein translated from the gene is a protein variant that has an amino acid sequence different from the amino acid sequence before mutation by the conservative substitution and/or modification of one or more amino acids at the N-terminus, C-terminus of and/or within the amino acid sequence, but retains the functions or properties of the protein before mutation. As used herein, the term "conservative substitution" means substituting one amino acid with another amino acid having similar structural and/or chemical properties. The conservative substitution may have little or no impact on the activity of the protein or polypeptide. The amino acid is selected from among alanine (Ala, A), isoleucine (Ile, I), valine (Val, V), leucine (Leu, L), methionine (Met, M), asparagine (Asn, N), cysteine (Cys, C), glutamine (Gln, Q), serine (Ser, S), threonine (Thr, T), phenylalanine (Phe, F), tryptophan (Trp, W), tyrosine (Tyr, Y), aspartic acid (Asp, D), glutamic acid (Glu, E), arginine (Arg, R), histidine (His, H), lysine (Lys, K), glycine (Gly, G), and proline (Pro, P).

In addition, some variants include those in which one or more portions, such as an N-terminal leader sequence or transmembrane domain, have been removed, or those in which a portion has been removed from the N- and/or C-terminus of a mature protein.

The variant may have increased (enhanced), unchanged, or decreased (weakened) ability compared to that of the protein before mutation. Here, the term "increased or enhanced" includes: a case in which the activity of the protein itself has increased compared to the activity of the protein before mutation; a case in which the overall activity of the protein in the cell is higher than that in the wild-type strain or the strain expressing the protein before mutation due to increased expression or translation of the gene encoding the protein; and a combination thereof. In addition, the term "decreased or weakened" includes: a case in which the activity of the protein itself has decreased compared to the activity of the protein before mutation; a case in which the overall activity of the protein in the cell is lower than that in the wild-type strain or the strain expressing the protein before mutation due to reduced expression or translation of the gene encoding the protein; and a combination thereof. In the present invention, the term "variant" may be used interchangeably with terms such as variant type, modification, variant polypeptide, mutated protein, mutation, and the like.

The alkylhydroperoxidase AhpD family core domain variant according to the present invention may comprise an amino acid sequence having at least 70%, 75%, 80%, 85%, 90%, 95%, 96%, 97%, 98%, 99% or 100% homology or identity to the amino acid sequence of SEQ ID NO: 4, except for the mutation position (position 231), and may comprise, without limitation, any amino acid sequence that retains the function or characteristic of the variant.

Another aspect of the present invention provides a polynucleotide encoding the alkylhydroperoxidase AhpD family core domain variant.

As used in the present invention, the term "polynucleotide" refers to a DNA or RNA strand having a certain length or more, which is a long-chain polymer of nucleotides formed by linking nucleotide monomers via covalent bonds. Specifically, the term "polynucleotide" refers to a polynucleotide fragment encoding the alkylhydroperoxidase AhpD family core domain variant.

According to one embodiment of the present invention, the polynucleotide may comprise a nucleotide sequence encoding the amino acid sequence of SEQ ID NO: 4. For example, it may comprise the nucleotide sequence of SEQ ID NO: 3.

Still another aspect of the present invention provides a vector comprising a polynucleotide encoding the alkylhydroperoxidase AhpD family core domain variant.

Yet another aspect of the present invention provides a transformant comprising the alkylhydroperoxidase AhpD family core domain variant or the polynucleotide.

As used in the present invention, the term "vector" refers to any type of nucleic acid sequence transfer structure that is used as a means for transferring and expressing a gene of interest in a host cell. Unless otherwise specified, the term "vector" may mean one allowing the nucleic acid sequence contained therein to be expressed after insertion into the host cell genome and/or one allowing the nucleic acid sequence to be expressed independently. This vector comprises essential regulatory elements operably linked so that an inserted gene can be expressed. As used herein, the term "operably linked" means that a gene of interest and regulatory sequences thereof are functionally linked together in a manner enabling gene expression, and the "regulatory sequences" include a promoter sequence for initiating transcription, any operator sequence for regulating transcription, a sequence encoding suitable mRNA ribosome-binding sites, and a sequence for regulating termination of transcription and translation.

The vector that is used in the present invention is not particularly limited as long as it may replicate in a host cell, and any vector known in the art may be used. Examples of the vector include a natural or recombinant plasmid, cosmid, virus and bacteriophage. Examples of a phage vector or cosmid vector include, but are not limited to, pWE15, M13, AMBL3, AMBL4, λIXII, AASHII, λAPII, λt10, At11, Charon4A, and Charon21A, and examples of a plasmid vector include, but are not limited to, pBR series, pUC series, pBluescriptII series, pGEM series, pTZ series, pCL series, and pET series.

The vector may typically be constructed as a vector for cloning or as a vector for expression. The vector for expression may be a conventional vector that is used in the art to express a foreign gene or protein in a plant, animal, or microorganism, and may be constructed through various methods known in the art.

The "recombinant vector" used in the present invention may be constructed using a prokaryotic or eukaryotic cell as a host, and may replicate regardless of the genome of the host cell or may be integrated into the genome itself. The host cell may contain a replication origin, which is a particular nucleotide sequence which enables the vector to replicate in the host cell and from which replication starts. For example, when the vector used is an expression vector and uses a prokaryotic cell as a host, the vector generally comprises a strong promoter capable of promoting transcription (e.g., pLλ promoter, CMV promoter, trp promoter, lac promoter, tac promoter, and T7 promoter), a ribosome binding site for initiation of translation, and a transcription/translation termination sequence. When a eukaryotic cell is used as a host, the vector comprises a replication origin operating in the eukaryotic cell, and examples of the replication origin include, but are not limited to, an f1 replication origin, an SV40 replication origin, a pMB1 replication origin, an adeno replication origin, an AAV replication origin, and a BBV replication origin. In addition, the vector may comprise a promoter derived from the genome of a mammalian cell (e.g., metallothionein promoter) or a promoter derived from a mammalian virus (e.g., adenovirus late promoter, vaccinia virus 7.5K promoter, SV40 promoter, cytomegalovirus promoter, and HSV-tk promoter), and generally has a polyadenylation sequence as a transcription termination sequence.

The recombinant vector may comprise a selection marker. The selection marker serves to select a transformant (host cell) transformed with the vector, and since only cells expressing the selection marker can survive in the medium treated with the selection marker, it is possible to select transformed cells. Representative examples of the selection marker include, but are not limited to, kanamycin, streptomycin, and chloramphenicol.

The transformant may be produced by inserting the recombinant vector into a host cell, and the transformant may be obtained by introducing the recombinant vector into an appropriate host cell. The host cell is a cell capable of stably and continuously cloning or expressing the expression vector, and any host cell known in the art may be used.

Where the vector is transformed into prokaryotic cells to generate recombinant microorganisms, examples of host cells that may be used include, but are not limited to, *E. coli* sp. strains such as *E. coli* DH5α, *E. coli* JM109, *E. coli* BL21, *E. coli* RR1, *E. coli* LE392, E. *coli B, E. coli* X 1776, *E. coli* W3110, and *E. coli* XL1-Blue, *Corynebacterium* sp. strains, *Bacillus* sp. strains such as *Bacillus subtilis* and *Bacillus thuringiensis,* and various Enterobacteriaceae strains such as *Salmonella typhimurium, Serratia marcescens,* and *Pseudomonas* species.

Where the vector is transformed into eukaryotic cells to generate recombinant microorganisms, examples of host cells that may be used include, but are not limited to, yeast (e.g., *Saccharomyces cerevisiae*)*,* insect cells, and plant cells and animal cells, such as Sp2/0, CHO K1, CHO DG44, PER.C6, W138, BHK, COS7, 293, HepG2, Huh7, 3T3, RIN, and MDCK cell lines.

As used in the present invention, the term "transformation" refers to a phenomenon in which external DNA is introduced into a host cell, thereby artificially causing genetic changes, and the term "transformant" refers to a host cell into which external DNA has been introduced and in which the expression of the gene of interest is stably maintained.

The transformation may be performed using a suitable vector introduction technique selected depending on the host cell, so that the gene of interest or a recombinant vector comprising the same may be expressed in the host cell. For example, introduction of the vector may be performed by electroporation, heat-shock, calcium phosphate (CaPO₄) precipitation, calcium chloride (CaCl₂) precipitation, microinjection, polyethylene glycol (PEG) method, DEAE-dextran method, cationic liposome method, lithium acetate-DMSO method, or any combination thereof, without being limited thereto. As long as the transformed gene may be expressed in the host cell, it may be inserted into the chromosome of the host cell, or may exist extrachromosomally, without being limited thereto.

The transformant may include a cell transfected, transformed, or infected with the recombinant vector of the present invention *in vivo* or *in vitro,* and may be used in the same sense as a recombinant host cell, a recombinant cell, or a recombinant microorganism.

According to one embodiment of the present invention, the transformant may be an *Escherichia* sp. strain or a *Corynebacterium* sp. strain.

The *Escherichia* sp. strain may be *Escherichia coli, Escherichia albertii, Escherichia blattae, Escherichia fergusonii, Escherichia hermannii,* or *Escherichia vulneris,* without being limited thereto.

The *Corynebacterium* sp. strain may be *Corynebacterium glutamicum, Corynebacterium crudilactis, Corynebacterium deserti, Corynebacterium callunae, Corynebacterium suranareeae, Corynebacterium lubricantis, Corynebacterium doosanense, Corynebacterium efficiens, Corynebacterium uterequi, Corynebacterium stationis, Corynebacterium pacaense, Corynebacterium singulare, Corynebacterium humireducens, Corynebacterium marinum, Corynebacterium halotolerans, Corynebacterium spheniscorum, Corynebacterium freiburgense, Corynebacterium striatum, Corynebacterium canis, Corynebacterium ammoniagenes, Corynebacterium renale, Corynebacterium pollutisoli, Corynebacterium imitans, Corynebacterium caspium, Corynebacterium testudinoris, Corynebacterium pseudopelargi,* or *Corynebacterium flavescens,* without being limited thereto.

For example, the transformant may be a *Corynebacterium* sp. strain, specifically *Corynebacterium glutamicum.*

The transformant in the present invention may be a strain either comprising the above-described alkylhydroperoxidase AhpD family core domain variant or a polynucleotide encoding the same or comprising the vector comprising the same, a strain expressing the alkylhydroperoxidase AhpD family core domain variant or the polynucleotide, or a strain having activity for the alkylhydroperoxidase AhpD family core domain variant, without being limited thereto.

According to one specific example of the present invention, the transformant may be obtained by transformation to express the alkylhydroperoxidase AhpD family core domain variant, or may be obtained by transformation through introduction of a polynucleotide encoding the alkylhydroperoxidase AhpD family core domain variant.

The transformant in the present invention may comprise other protein variants or genetic mutations, in addition to the alkylhydroperoxidase AhpD family core domain variant.

According to one embodiment of the present invention, the transformant may have the ability to produce L-glutamic acid.

The transformant may naturally have the ability to produce L-glutamic acid or may be one artificially endowed with the ability to produce L-glutamic acid.

According to one embodiment of the present invention, the transformant may have an increased ability to produce L-glutamic acid, due to a change in the activity of the alkylhydroperoxidase AhpD family core domain.

Specifically, it may be expected that the transformant according to the present invention has an increased ability to produce L-glutamic acid in the latter half of fermentation by an increased ability to respond to oxidative stress, which is due to a mutation in the alkylhydroperoxidase AhpD family core domain.

As used in the present invention, the term "increased ability to produce" means that L-glutamic acid productivity has increased compared to that of the parent strain. As used herein, the term "parent strain" refers to a wild-type strain or mutant strain to be mutated, and includes a strain that is to be mutated directly or to be transformed with a recombinant vector or the like. In the present invention, the parent strain may be a wild-type *Escherichia* sp. or *Corynebacterium* sp. strain or an *Escherichia* sp. or *Corynebacterium* sp. strain mutated from the wild-type strain.

The transformant according to the present invention exhibits an increased ability to produce L-glutamic acid compared to the strain (parent strain) containing the protein before mutation, due to the change in alkylhydroperoxidase AhpD family core domain activity caused by introduction of the alkylhydroperoxidase AhpD family core domain variant thereinto. Specifically, the amount of L-glutamic acid produced by the transformant may be at least 1%, 5%, 10%, 15%, 20%, 25%, 30%, 35%, 40%, 45%, 50%, 55%, 60%, 65%, 70%, 75%, 80%, 85%, 90%, 95%, or 100% higher than that produced by the parent strain, or may be 1.1-fold, 1.5-fold, 2-fold, 2.5-fold, 3-fold, 3.5-fold, 4-fold, 4.5-fold, 5-fold, 5.5-fold, 6-fold, 6.5-fold, 7-fold, 7.5-fold, 8-fold, 8.5-fold, 9-fold, 9.5-fold, or 10-fold, 20-fold, 30-fold, 40-fold, 50-fold, 60-fold, 70-fold, 80-fold, 90-fold, or 100-fold higher than that produced by the parent strain, without being limited thereto.

A composition comprising the transformant according to the present invention may be used as a composition for producing L-glutamic acid.

Still yet another aspect of the present invention provides a method for producing L-glutamic acid, comprising steps of: culturing the transformant in a medium; and recovering L-glutamic acid from the transformant or the medium in which the transformant has been cultured.

The culturing may be performed using a suitable medium and culture conditions known in the art, and any person skilled in the art may easily adjust and use the medium and the culture conditions. Specifically, the medium may be a liquid medium, without being limited thereto. Examples of the culturing method include, but are not limited to, batch culture, continuous culture, fed-batch culture, or a combination thereof.

According to one embodiment of the present invention, the medium should meet the requirements of a specific strain in a proper manner, and may be appropriately modified by a person skilled in the art. For culture media for *Escherichia* sp. or *Corynebacterium* sp. strains, reference may be made to, but not limited to, a known document (Manual of Methods for General Bacteriology, American Society for Bacteriology, Washington D.C., USA, 1981).

According to one embodiment of the present invention, the medium may contain various carbon sources, nitrogen sources, and trace element components. Examples of carbon sources that may be used include: sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture, without being limited thereto. Examples of nitrogen sources that may be used include peptone, yeast extract, meat extract, malt extract, corn steep liquor, soybean meal, urea, or inorganic compounds such as ammonium sulfate, ammonium chloride, ammonium phosphate, ammonium carbonate, and ammonium nitrate. The nitrogen sources may also be used individually or as a mixture, without being limited thereto. Examples of phosphorus sources that may be used include, but are not limited to, potassium dihydrogen phosphate or dipotassium hydrogen phosphate or the corresponding sodium-containing salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be used in the culture medium. The medium or individual components may be added to the culture medium batchwise or in a continuous manner by a suitable method during culturing, without being limited thereto.

According to one embodiment of the present invention, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present invention, in the step of recovering L-glutamic acid from the cultured transformant or the medium in which the transformant has been cultured, the produced L-glutamic acid may be collected or recovered from the medium using a suitable method known in the art depending on the culture method. Examples of a method that may be used to recover the produced L-glutamic acid include, but are not limited to, centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobicity and size exclusion), and the like.

According to one embodiment of the present invention, the step of recovering L-glutamic acid may be performed by centrifuging the culture medium at a low speed to remove biomass and separating the obtained supernatant through ion-exchange chromatography.

According to one embodiment of the present invention, the step of recovering L-glutamic acid may include a process of purifying the L-glutamic acid.

### Advantageous Effects

The alkylhydroperoxidase AhpD family core domain variant according to the present invention is obtained by substituting one or more amino acids in the amino acid sequence constituting the alkylhydroperoxidase AhpD family core domain to change the activity of the protein, and L-glutamic acid may be efficiently produced from a recombinant microorganism expressing the variant.

### Mode for Invention

Hereinafter, the present invention will be described in more detail. However, this description is merely presented by way of example to facilitate the understanding of the present invention, and the scope of the present invention is not limited by this exemplary description.

### Example 1. Construction of Strain Expressing Alkylhydroperoxidase AhpD Family Core Domain Variant

To evaluate the effect of a variant (SEQ ID NO: 4) having a substitution of arginine (R) for tryptophan (W) at position 231 in the amino acid sequence (SEQ ID NO: 2) of the alkylhydroperoxidase AhpD family core domain on the production of L-glutamic acid, a vector for expressing the alkylhydroperoxidase AhpD family core domain variant and a strain into which the vector has been introduced were constructed.

### 1-1. Construction of Vector pK_ahpD1(W231R) for Transformation

Using the genomic DNA of *Corynebacterium glutamicum* ATCC13869 as a template, PCR reactions were performed using a primer pair of primers 1 and 2 and a primer pair of primers 3 and 4, respectively. Two PCR products of about 0.5 kb and 0.6 kb in size amplified through PCR were mixed and used as templates, and overlapping PCR was performed using a primer pair of primers 1 and 4 to obtain a single fragment. A pK19msb vector (SEQ ID NO: 5) were treated with the restriction enzyme smaI (NEB) and then ligated with the fragment using T4 ligase. The resulting constructed vector was named pK_ahpD1(W231R). The PCR was performed using Pfu PreMix (Bioneer) under the following conditions: denaturation at 95°C for 5 min, and then 30 cycles, each consisting of 95°C for 30 sec, 55°C for 30 sec, and 72°C for 1 min, followed by reaction at 72°C for 5 min. The primer sequences used for vector construction are shown in Table 1 below.

**[Table 1]**

| SEQ ID NO. | Primer name | Primer sequence (5'→3') |
|---|---|---|
| 6 | Primer 1 | CGCAAACCGATTTCACTCGC |
| 7 | Primer 2 | CCATGGCTTCCGGCCCAAAGAG |
| 8 | Primer 3 | CTCTTTGGGCCGGAAGCCATGG |
| 9 | Primer 4 | AGAGCTTGGCCAGTAAACGG |

### 1-2. Construction of L-Glutamic Acid-Producing Strain into Which pK_ahpD1(W231R) Has Been Introduced

Corynebacterium glutamicum U3 (accession number: KCCM13218P) was used as a parent strain to produce an L-glutamic acid-producing strain. The pK_ahpD1(W231R) vector was prepared to a final concentration of 1 µg/µl or more, and electroporated into *Corynebacterium glutamicum* U3 (see Tauch et al., FEMS Microbiology letters 123 (1994) 343-347). Then, 1 ml of a regeneration medium (containing 18.5 g/l brain heart infusion and 91 g/l sorbitol) was added to the cells which were then heat-treated at 46°C for 6 minutes. After treatment, the cells were transferred into a 15-ml cap tube, incubated at 30°C for 2 hours, and plated on a selection medium (containing 5 g/l tryptone, 5 g/l NaCl, 2.5 g/l yeast extract, 18.5 g/l brain heart infusion powder, and 15 g/l agar) containing 20 mg/l kanamycin. The cells were cultured at 30°C for 72 hours, and the generated colonies were cultured in BHI medium (18.5 g/l brain heart infusion powder) for 15 hours to induce secondary recombination. Then, the cells were diluted to 10⁻² to 10⁻³, and plated on a selection medium containing 10% sucrose, and the colonies were isolated. The isolated colonies were cultured on two types of selective media supplemented with kanamycin and sucrose, respectively, and a strain that had no kanamycin resistance and could grow on the medium containing sucrose was selected. The selected strain was named ahpD1(W231R).

### Experimental Example 1. Evaluation of L-Glutamic Acid Productivity

The L-glutamic acid productivity of the strain ahpD1(W231R) expressing the alkylhydroperoxidase AhpD family core domain variant was evaluated compared to that of the parent strain *Corynebacterium glutamicum* U3.

Each strain was inoculated at 1% by volume into a 100-mL flask containing 10 mL of the medium for glutamic acid production shown in Table 2 below, and cultured with shaking at 200 rpm at 30°C for 48 hours. After completion of the culturing, the concentration of L-glutamic acid in the medium was measured using HPLC (Agilent), and the results are shown in Table 3 below.

**[Table 2]**

| Component | Medium for L-glutamic acid production |
|---|---|
| Glucose | 70 g/L |
| (NH₄)₂SO₄ | 5 g/L |
| MgSO₄ | 0.4 g/L |
| Urea | 2 g/L |
| Soybean hydrolyzate | 15 ml/L |
| KH₂PO₄ | 1 g/L |
| FeSO₄ | 10 mg/L |
| MnSO₄ | 10 mg/L |
| Thiamine_HCl | 200 ug/L |
| Biotin | 2 ug/L |
| CaCO₃ | 5.0% |

**[Table 3]**

| | L-glutamic acid production (g/L) |
|---|---|
| Parent strain | 15.3 |
| ahpD1(W231R) | 16.9 |

As shown in Table 3 above, it was confirmed that the strain ahpD1(W231R) expressing the alkylhydroperoxidase AhpD family core domain variant showed an increase of about 10.4% in L-glutamic acid production compared to the parent strain due to the substitution of arginine for amino acid tryptophan at position 231 in the amino acid sequence of the alkylhydroperoxidase AhpD family core domain.

So far, the present invention has been described with reference to the preferred embodiments. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

### [Accession Number]

Depository Authority: Korean Culture Center of Microorganisms (KCCM)
Accession Number: KCCM13218P
Deposit Date: June 29, 2022

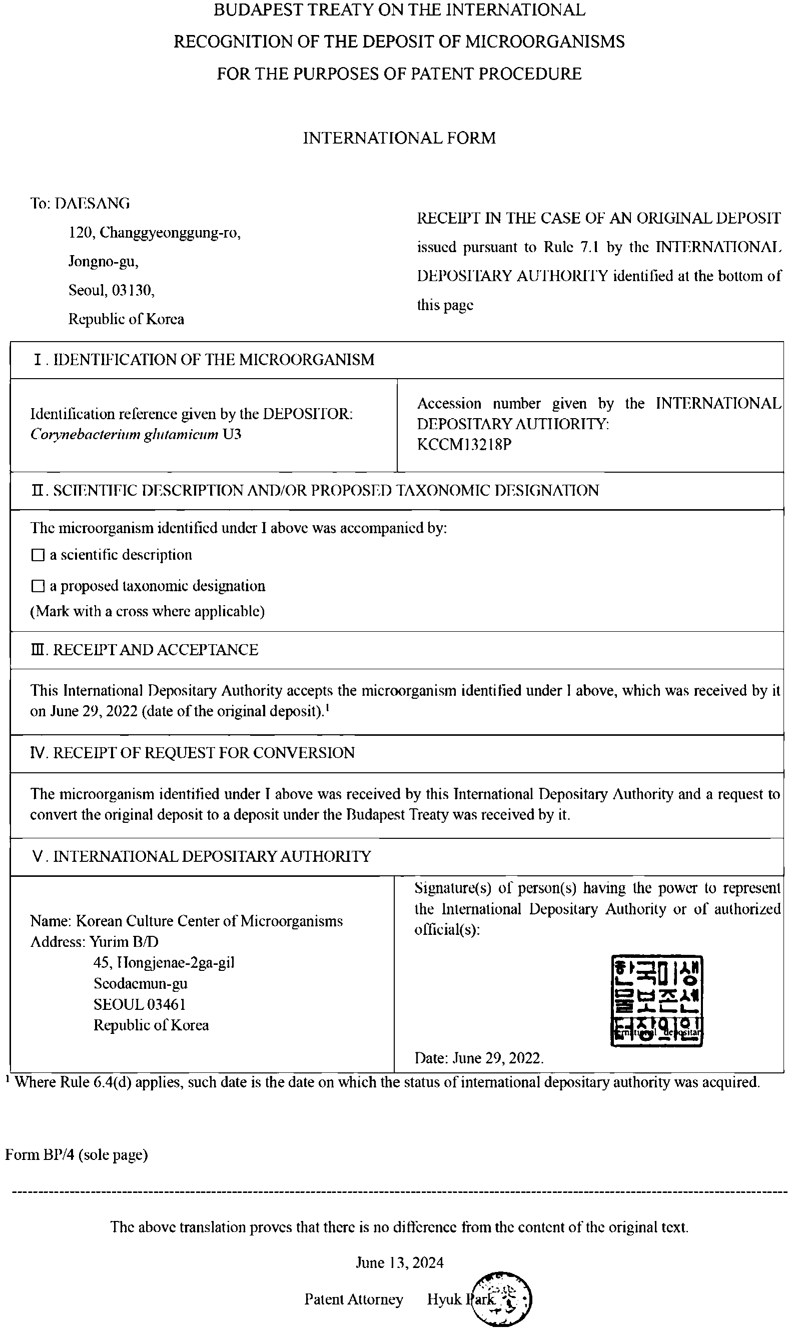
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To: DAESANG
120, Changgyeonggung-ro,
Jongno-gu,
Seoul, 03130,
Republic of Korea

RECEIPT IN THE CASE OF AN ORIGINAL DEPOSIT
issued pursuant to Rule 7.1 by the INTERNATIONAL
DEPOSITARY AUTHORITY identified at the bottom of
this page

I. IDENTIFICATION OF THE MICROORGANISM
Identification reference given by the DEPOSITOR:
Corynebacterium glutamicum U3 | Accession number given by the INTERNATIONAL
DEPOSITARY AUTHORITY:
KCCM13218P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above, which was received by it
on June 29, 2022 (date of the original deposit).¹

IV. RECEIPT OF REQUEST FOR CONVERSION
The microorganism identified under I above was received by this International Depositary Authority and a request to
convert the original deposit to a deposit under the Budapest Treaty was received by it.

V. INTERNATIONAL DEPOSITARY AUTHORITY
Name: Korean Culture Center of Microorganisms
Address: Yurim B/D
45, Hongjenae-2ga-gil
Seodaemun-gu
SEOUL 03461
Republic of Korea | Signature(s) of person(s) having the power to represent
the International Depositary Authority or of authorized
official(s):
Date: June 29, 2022.

¹ Where Rule 6.4(d) applies, such date is the date on which the status of international depositary authority was acquired.

Form BP/4 (sole page)

The above translation proves that there is no difference from the content of the original text.

June 13, 2024

Patent Attorney Hyuk Park

## Claims

1. An alkylhydroperoxidase AhpD family core domain variant consisting of the amino acid sequence of SEQ ID NO: 4 in which tryptophan (W) at position 231 in the amino acid sequence of SEQ ID NO: 2 is substituted with arginine (R).

2. A polynucleotide encoding the variant of claim 1.

3. A transformant comprising the variant of claim 1 or the polynucleotide of claim 2.

4. The transformant of claim 3, which is an *Escherichia* sp. strain or a *Corynebacterium* sp. strain.

5. The transformant of claim 3, which has ability to produce L-glutamic acid.

6. A method for producing L-glutamic acid, comprising steps of:
culturing the transformant of claim 3 in a medium; and
recovering L-glutamic acid from the transformant or the medium in which the transformant has been cultured.
